# EUROPÄISCHE PATENTSCHRIFT

(11) **EP 1 246 696 B1**
(45) Veröffentlichungstag und Bekanntmachung des Hinweises auf die Patenterteilung: **11.10.2006**
(21) Anmeldenummer: 00989971.7
(22) Anmeldetag: 11.12.2000
(51) Int. Cl.: B01J 23/34

(54) **OXIDATIONSREAKTIONEN UNTER VERWENDUNG VON GEMISCHTLEITENDEN, SAUERSTOFFSELEKTIVEN MEMBRANEN**
OXIDATIVE REACTIONS USING MEMBRANES THAT SELECTIVELY CONDUCT OXYGEN
REACTIONS D'OXYDATION A L'AIDE DE MEMBRANES A CONDUCTION SELECTIVE DES IONS D'OXYGENE ET A CONDUCTION SIMULTANEE DES ELECTRONS

(30) Priorität: 10.12.1999 DE 19959873
(43) Veröffentlichungstag der Anmeldung: 09.10.2002
(73) Patentinhaber: BASF Aktiengesellschaft, 67056 Ludwigshafen (DE)
(72) Erfinder: BITTERLICH, Stefan, 67246 Dirmstein (DE); VOSS, Hartwig, 67227 Frankenthal (DE); HIBST, Hartmut, 69198 Schriesheim (DE); TENTEN, Andreas, 67487 Maikammer (DE); VOIGT, Ingolf, 07743 Jena (DE); PIPPARDT, Ute, 07639 Bad Klosterlausnitz (DE)
(74) Vertreter: Isenbruck, Günter
(86) Internationale Anmeldenummer: PCT/EP2000/012497
(87) Internationale Veröffentlichungsnummer: WO 2001/041924

(56) Entgegenhaltungen:
- EP-A- 0 641 749
- EP-A- 0 963 788
- US-A- 5 830 822
- US-A- 5 849 659

## Beschreibung

Die vorliegende Erfindung betrifft das Gebiet der heterogenen Katalyse. Genauer gesagt, bezieht sich die vorliegende Erfindung auf Oxidationsreaktionen, die an einem heterogenen Katalysator durchgeführt werden, der auf einer sogenannten gemischtleitfähigen Membran angebracht ist. Solche Membranen weisen eine selektive Leitfähigkeit für Sauerstoffionen und gleichzeitig Elektronenleitfähigkeit auf.

Sauerstoffpermeable Membranen, die einen selektiven Transport von Sauerstoff durch die Membranen hindurch ermöglichen, sind lange bekannt. Derartige Membranen gestatten es, Sauerstoff aus Gemischen dieses Elements mit anderen, vorzugsweise gasförmigen, Elementen abzutrennen. Die Selektivität beruht dabei auf der Wanderung von O²⁻-Ionen durch eine Schicht aus bestimmten keramischen Materialien. Bevor der Sauerstoff die Membran durchdringt, werden auf einer Seite der Membran Elektronen auf den Sauerstoff übertragen. Es erfolgt dann die Wanderung, auf der anderen Seite der Membran werden den Oxidionen die Elektronen wieder entzogen, und der Sauerstoff wird frei. Bei den erwähnten keramischen Materialien handelt es sich etwa um oxidische Verbindungen von Übergangsmetallen. Diese müssen, um den Sauerstoffionentransport zu ermöglichen, kristallographische Leerstellen im Gitter aufweisen.

Diese Leerstellen im Gitter lassen sich beispielsweise erzeugen durch Ersetzen von Metallionen in einem gegebenen Oxid durch Ionen einer anderen Wertigkeit. So entstehen auf eine relativ einfache Art und Weise sogenannte O²⁻-Leerstellen, über die der Transport der Oxidionen zum anderen Ende der Membran verläuft.

Das bestbekannte sauerstoffselektive keramische Material ist dabei ZrO₂, bei dem die Oxidionenleitfähigkeit durch einen teilweisen Ersatz des vierwertigen Zirkoniums durch dreiwertige Yttrium- oder zweiwertige Calcium-Ionen erzeugt wird. Wird ein solches Material als sauerstoffselektive Membran verwendet, so wird ein Material erhalten, das den Transport von O²⁻-Ionen ermöglicht. Das Material weist keine Elektronenleitfähigkeit auf. Ein solches Material ist somit als solches allein nicht zum Transport von Sauerstoff befähigt. Wenn Sauerstoff durch eine Membran aus einem solchen Material transportiert werden soll, dann muß durch Anlegen eines äußeren Stromkreises an beiderseitig der Membran aufgebrachte Elektroden ein Ladungsausgleich herbeigeführt werden.

Um das Anlegen des äußeren Stromkreises überflüssig zu machen, wurde vorgeschlagen, Membranen herzustellen, die neben der Oxidionenleitfähigkeit auch eine Elektronenleitfähigkeit aufweisen. Dieses wird generell erreicht, indem eine zweite Komponente mit einer guten Elektronenleitfähigkeit der keramischen Membran beigefügt wird. Häufig wird dafür auf Palladium, Platin, Silber oder Gold bzw. leitfähige oxidische Verbindungen dieser oder anderer Metalle zurückgegriffen. Es entstehen so zweiphasige Membranen, sogenannte Dual-Phase-Membranen. Diese werden beispielsweise in der EP-A-399 833 offenbart. Darin werden unter anderem sauerstoffselektive, elektronenleitende Membranen beschrieben, die aus einem Gemisch von 25 bis 99 Vol.-% von Zirkoniumdioxid, das mit Yttrium dotiert ist, und 1 bis 75 Vol.-% Platin bestehen. Solche Membranen werden dann in Oxidationsreaktionen eingesetzt, bei denen der Sauerstoff aus Gemischen durch die Membran selektiv abgetrennt und der Reaktion zugeführt wird.

Eine Weiterentwicklung der vorstehend beschriebenen Membranen stellen die sogenannten gemischtleitenden Membranen dar. Diese bestehen nicht aus zwei verschiedenen Materialien bzw. Phasen, sondern einem einzigen Material, das die Eigenschaft aufweist, selektiv sowohl Oxidionen als auch Elektronen zu leiten. Diese Leitereigenschaften finden sich nur bei einer stark begrenzten Anzahl von Materialien. Im allgemeinen werden bestimmte Perowskite verwendet, die generell mit anderen Kationen dotiert sind, um die Leitfähigkeitseigenschaften und die Temperaturbeständigkeit zu erhöhen.

Im Stand der Technik finden sich einige Veröffentlichungen, die solche Materialien und damit hergestellte gemischtleitende Membranen beschreiben.

Die beiden Patente US 4,791,079 und US 4,827,071 beschreiben gemischtleitende, oxidionenselektive Membranen, die aus Zirkoniumdioxid bestehen, das mit einem Metall der Gruppe VB, VIB oder Titandioxid dotiert ist. Das bevorzugt verwendete Keramikmaterial ist dabei mit Yttriumoxid und Titandioxid versehenes Zirkoniumdioxid. Auf der Membran ist dann ein poröses Material angebracht, das einen Oxidationskatalysator für Kohlenwasserstoffe enthält. Je nach Art dieses Katalysators lassen sich die so erhaltenen Membranen in verschiedenen Oxidationsreaktionen einsetzen, beispielsweise der Herstellung von Ethylenoxid oder Propylenoxid aus dem jeweiligen Olefin, der oxidativen Dehydrierung vom Monoloefinen, oder der oxidativen Kopplung von Methan oder anderen Alkanen.

In der EP-A-663 232 findet sich die Beschreibung einer gemischtleitenden, oxidionenselektiven Membran, die aus einem mehrere verschiedene Metallionen aufweisenden Metalloxid und einem darauf angebrachten Katalysator besteht. Vorzugsweise weist das Oxid Wismut-, Barium-, Vanadium-, Molybdän-, Cer-, Ruthenium-, Mangan-, Kobalt-, Rhodium- oder Praseodym-Ionen auf. Der Katalysator ist vorzugsweise ein Metall aus der Gruppe, die aus Platin, Palladium, Gold und Silber gebildet wird. Mit den so erhaltenen Membrankatalysatoren lassen sich verschiedene Oxidationen durchführen, beispielsweise die Konvertierung von Methan zu Sythesegas, aber auch die Überführung von Oxiden von beispielsweise Schwefel oder Stickstoff in die jeweiligen Elemente, beispielsweise die Umwandlung von Schwefeloxiden in Schwefel und Sauerstoff.

Die WO 98/41394 lehrt eine oxidionenselektive Membran der allgemeinen Formel ABO_{3-δ}, worin A und B ausgewählt sind aus Calcium, Strontium, Barium, Yttrium und Lanthan (A), bzw. Chrom, Mangan, Eisen, Kobalt, Nickel und Kupfer, und δ eine Zahl zwischen 0 und 0,5 ist. Die Membran ist auf beiden Seiten mit einem Katalysator versehen, nämlich auf der einen Seite mit einem Katalysator zur Aktivierung von Sauerstoff, und auf der anderen Seite mit einem Katalysator zur Partialoxidation von Kohlenwasserstoffen. Mit einem Katalysator einer solchen Zusammensetzung läßt sich beispielsweise selektiv die Teiloxidation von Methan zu Kohlenmonoxid und Wasserstoff durchführen.

Schließlich findet sich in der WO 99/21649 die Beschreibung einer Reaktionsmembran, die aus einer gemischtleitenden, oxidionenselektiven Membran besteht, auf der eben der Oxidationskatalysator angebracht ist. Die Membran ist eine oxidische Verbindung von mehreren Metallen, die ausgewählt sind aus der Gruppe, die aus Lanthaniden, Yttrium, 3d-Übergangsmetallen und Metallen der 13. Gruppe besteht. Eine Fülle von Oxidationskatalysatoren, sowohl oxidischen als auch metallischen, die auf solchen Membranen angebracht werden können, sind offenbart.

Alle die in den oben zitierten Referenzen erwähnten Oxidationsreaktionen werden jedoch generell bei hohen Temperaturen, im allgemeinen ca. 800 bis 1000°C, durchgeführt. Dies liegt daran, daß mit den bisher im Stand der Technik erwähnten gemischtleitenden, oxidionenselektiven Membranen lediglich bei solchen Temperaturen ein Sauerstoffdurchsatz erreicht werden kann, der eine industrielle Anwendung erlaubt.

Unterhalb des erwähnten Temperaturbereichs ist der Sauerstoffdiffusionskoeffizient der oben offenbarten Materialien so gering, daß nicht genügend Sauerstoff durch die Membran wandert. Der Einsatz dieser gemischtleitenden Membranen in Oxidationsreaktionen ist somit im allgemeinen auf solche Reaktionen beschränkt, die in dem erwähnten Temperaturbereich ablaufen. Es wäre jedoch wünschenswert, über auf gemischtleitenden, oxidionenselektiven Membranen angebrachte Oxidationskatalysatoren zu verfügen, die auch bei relativ niedrigen Temperaturen eingesetzt werden können.

Die Aufgabe der vorliegenden Erfindung ist somit die Bereitstellung einer solchen Reaktionsmembran. Dabei sollen die bisher bekannten Vorteile von auf gemischtleitenden Membranen angebrachten Oxidationskatalysatoren beibehalten werden. Dies ist insbesondere der Entfall der Notwendigkeit einer Luftzerlegung, in den Fällen, in denen die jeweilige Oxidationsreaktion mit reinem Sauerstoff durchgeführt werden muß. Außerdem wird oftmals die Selektivität der Oxidation durch Bereitstellung des Sauerstoffs als Gittersauerstoff, der an der Grenzfläche Katalysator/Reaktionsgas zur Verfügung steht, erhöht. Homogen in der Gasphase ablaufende, unerwünschte Nebenreaktionen werden unterdrückt.

Diese Aufgabe wird gelöst durch eine Reaktionsmembran enthaltend einen selektiven Oxidationskatalysator auf einer gemischtleitenden, oxidionenselektiven Keramikmembran der Zusammensetzung (Sr₁₋ₓCaₓ)_{1-y}A_{y}Mn_{1-z}B_{Z}O_{3-δ}, wobei
A für Ba, Pb, Na, K, Y oder Elemente der Lanthanidengruppe oder eine Kombination dieser Elemente steht,
B für Mg, Al, Ga, In, Sn, oder Elemente der 3d-Reihe oder der 4d-Reihe oder eine Kombination dieser Elemente steht,
x eine Zahl von 0,2 bis 0,8,
y eine Zahl von 0 bis 0,4,
z eine Zahl von 0 bis 0,6 und
δ eine Zahl ist, die sich in Abhängigkeit von x, y oder z aus der Einhaltung des Elektroneutralitätsprinzips ergibt.

Diese Aufgabe wird weiterhin gelöst durch die Verwendung der vorstehend beschriebenen Reaktionsmembran in Oxidationsreaktionen von Kohlenwasserstoffen unter Verwendung von Sauerstoff.

Die erfindungsgemäße Reaktionsmembran bzw. deren Verwendung in Oxidationsreaktionen gestattet die Durchführung dieser Reaktionen bei Temperaturen, bei denen dies bis jetzt nicht möglich war. Dieser Temperaturbereich liegt dabei insbesondere bei Werten von 350 bis 450°C. Ein entscheidender Punkt der erfindungsgemäßen Reaktionsmembran ist dabei die gemischtleitende, oxidionenselektive Membran.

Geeignete gemischtleitende Membranen sind dabei diejenigen, die in der deutschen Anmeldung Aktenzeichen 198 26 496.8 offenbart sind. Es handelt sich dabei um Oxide, die von β-SrMnO₃, einem Perowskit mit ABO₃-Struktur, abgeleitet sind. Diesem SrMnO₃ werden zur Erhöhung der Temperaturstabilität und der Oxidionendurchlässigkeit bestimmte Fremdionen zugesetzt.

Zum einen ist dies ein Kation, das einen kleineren Ionenradius als Strontium aufweist. Wird eine solche Modifikation des Basismaterials durchgeführt, so wird in jedem Fall Ca²⁺ zugefügt, so daß eine Verbindung der Zusammensetzung Sr₁₋ₓCaₓMnO_{3-δ} resultiert. Gegebenenfalls kann ein weiteres Ion, das kleiner ist als Strontium und das oben mit A bezeichnet wurde, eingebaut werden. Dieses weitere Ion A ist ausgewählt aus der Gruppe bestehend aus Ba, Pb, Na, K oder Y. Es ergeben sich danach Verbindungen der allgemeinen Formel (Sr₁₋ₓCaₓ)_{1-y}A_{y}MnO_{3-δ}, wobei x, y und δ wie oben definiert sind.

Weiterhin kann Mangan ersetzt werden durch ein Kation mit einem größeren Ionenradius, das nachfolgend als B bezeichnet wird. B kann ein Element der 3. Hauptgruppe, vorzugsweise Al³⁺, Ga³⁺, In³⁺, oder ein Element der 3d- und 4d-Reihe sein, beispielsweise Fe³⁺, Co²⁺ oder Ni²⁺. Ein besonderer Vorteil des Einbaus eines Metalls mit einer Wertigkeit <4 ist der, daß eine höhere Leitfähigkeit des Materials resultiert. Beim Einbau eines Kations B entstehen Verbindungen der allgemeinen Formel SrMn_{1-z}B_{z}O_{3-δ}, wobei z und δ wie oben definiert sind.

In einer weiteren Modifikation lassen sich Strontium und Mangan gleichzeitig ersetzen. Dabei entstehen Verbindungen der allgemeinen Formel (Sr₁₋ₓCaₓ)_{1-y}A_{y}Mn_{1-z}B_{z}O_{3-δ}, wobei x, y, z und δ wie oben definiert sind.

Ein Vorteil der derartigen gemischtleitenden Membranen liegt darin, daß bereits ab ca. 220°C O₂-Diffusionskoeffizienten von ungefähr 10⁻⁷ cm²/s erreicht werden. Dadurch wird es technisch interessant, derartige Membranen als Bestandteil von Reaktionsmembranen für selektive katalytische Oxidationen einzusetzen. Die bisher im Stand der Technik beschriebenen gemischtleitenden Membranen weisen Anwendungstemperaturen >700°C auf, die für solche selektiven katalytischen Oxidationen deutlich zu hoch sind.

Die beschriebenen Membranen können noch andere Materialien enthalten, die Oxidionen oder Elektronen leiten. Um die vorteilhaften Eigenschaften der Membran zu erhalten, sollte diese jedoch mehr als 10 Vol.-% des erfindungsgemäßen Keramikmaterials ausmachen. Weiterhin kann die beschriebene Membran auf einem porösen Trägermaterial angebracht sein, dessen Porenanteil im Bereich von 10 bis 85 % liegt.

Die selektiven Oxidationskatalysatoren, die erfindungsgemäß auf der vorstehend beschriebenen Membran angebracht werden können, sind solche, die in den allgemein bekannten Oxidationsreaktionen von Kohlenwasserstoffen verwendet werden. Beispiele für solche Oxidationsreaktionen sowie die dabei verwendeten Katalysatoren werden nachfolgend gegeben.

### Ammonoxidation von Propan zu Acrylnitril

Es werden beispielsweise Katalysatoren der Zusammensetzung Mo-V-Te-X-O verwendet, wobei X für Nb, Ta, W, Ti, Al, Zr, Cr, Mn, Fe, Ru, Co, Rh, Ni, Pd, Pt, Sb, Be, B, In, Ce steht. Eine Beschreibung von Katalysatoren dieses Typs findet sich in der EP-A-603 836.

### Ammonoxidation von Propen zu Acrylnitril

Es lassen sich beispielsweise Mo-, Bi- und Fe-haltige Katalysatoren verwenden. Diese sind beispielsweise in der EP-A-807 622 beschrieben.

### Ammonoxidation von o-m-p-Xylol

Es lassen sich beispielsweise V- und Sb-haltige Katalysatoren verwenden. Derartige Katalysatoren sind beispielsweise in der EP-A-222 249 beschrieben.

### Oxidation von n-Butan n-Buten oder Butadien zu Maleinsäureanhydrid

Es können oxidische Vanadin- und Phosphor-haltige Katalysatoren verwendet werden, wie sie etwa in der EP-A-646 045 beschrieben sind.

### Oxidation von o-Xylol zu Phthalsäureanhydrid

Es werden beispielsweise oxidische Vanadin- und Titan-haltige Katalysatoren verwendet, die beispielsweise in der DE-A-197 07 943 beschrieben sind.

### Ammonoxidation von o-Xylol zur Phthalsäuredinitril

Es lassen sich beispielsweise V- und Sb-haltige Katalysatoren verwenden, wie dies beispielsweise in der EP-A-222 249 beschrieben ist.

### Oxidation von Propen zu Acrolein

Hierbei lassen sich oxidische Molybdän-, Wismut- und Eisen-haltige Katalysatoren verwenden, wie dies beispielsweise in der EP-A-575 897 offenbart ist.

### Gasphasenoxidation von Acrolein zu Acrylsäure

Es werden vorteilhafterweise Mo- und V-haltige Katalysatoren verwendet, beispielsweise diejenigen, die in der EP-A-17 000 oder der EP-A-774 297 offenbart sind.

### Oxidation von Methacrolein zu Methacrylsäure

Es werden Cäsium-, Molybdän-, Phosphor- und Vanadin-haltige Katalysatoren verwendet. Dies ist beispielsweise in der EP-A-668 103 beschrieben.

### Oxidative Dehydrierungen

Es lassen sich dabei generell alle bekannten Oxidehydrierungskatalysatoren verwenden.

Bei der Dehydrierung von Propan zu Propen werden etwa oxidische Molybdän- und Kobalt-haltige Katalysatoren verwendet. Dies ist etwa in der Anmeldung WO 99/42404 beschrieben.

Bei der Dehydrierung von Butenen zu Butadien lassen sich etwa oxidische Antimon- und Eisen-haltige Katalysatoren verwenden. Dies ist etwa in der US 3,445,521 offenbart.

Bei der Dehydrierung von Ethylbenzol zu Styrol lassen sich alle bekannten Oxidehydrierungskatalysatoren verwenden.

Bei der oxidativen Dehydrierung von Methanol zu Formaldehyd werden oxidische Eisen- und Molybdän-haltige Katalysatoren verwendet, wie dies beispielsweise in der DE-A-24 42 311, der EP-A-423 692 oder der EP-A-591 572 offenbart ist.

Der auf der Membran angebrachte Oxidationskatalysator ist im einfachsten Fall so beschaffen, daß er porös ist und/oder auf ein poröses Material aufgebracht ist. In einer bevorzugten Ausführungsform ist der Oxidationskatalysator aus einem Material, das selbst gemischtleitend und oxidionenselektiv ist. Der Vorteil einer solchen Ausführungsform liegt darin, daß der Katalysator in diesem Fall ohne durchgehende Poren auf die eigentlich gemischtleitende Membran aufgebracht werden kann. Dadurch wird das Reaktionsgas gegebenenfalls vor einer unerwünschten katalytischen Einwirkung des Membranmaterials geschützt.

Die Dicke der Katalysatorschicht beträgt von 10 Å bis 1 mm. Die Katalysatorschicht kann beispielsweise asymmetrisch derart aufgebaut sein, daß die Zone, die an die gemischtleitende Schicht angrenzt, porenfrei ist. Daran schließt sich dann eine Zone an, die eine zum Reaktionsgemisch hin offene Porosität aufweist.

In einer bevorzugten Ausführungsform der vorliegenden Erfindung befindet sich zwischen der gemischtleitenden Membran und dem Oxidationskatalysator eine oxidische Zwischenschicht mit einer Dicke von 10 Å bis 10 µm. Diese Schicht dient zum Erhöhen der mechanischen Festigkeit und/oder zur Erleichterung des Durchtritts der geladenen Spezies von der Membran in die Katalysatorschicht und umgekehrt.

Die erfindungsgemäße Reaktionsmembran enthaltend den Oxidationskatalysator kann verschieden gestaltet sein. In einer Ausführungsform ist die Membran flach, wobei der Katalysator auf einer Seite der Membran angebracht ist. In einer anderen Ausführungsform weist die Membran eine Rohrgeometrie mit Durchmessern von 4 bis 100 mm auf. Je nach vorgesehenem Einsatzzweck ist die Katalysatorschicht dabei auf der Außen- oder Innenseite angebracht. Selbstverständlich ist die Form der Reaktionsmembran mit dem Oxidationskatalysator nicht auf die oben beschriebenen Geometrien beschränkt, sondern die Membran kann auch noch in anderen, einem Fachmann bekannten Geometrien und Formen vorliegen.

Der Oxidationskatalysator wird auf die Membran mit den üblichen, einem Fachmann bekannten Methoden aufgebracht. Dies sind beispielsweise die Sol-Gel-Abscheidung, Sputtern, Fällen, Kristallisation, die sogenannte chemical vapor deposition oder physical vapor deposition, oder die Schlickerbeschichtung. Nach dem Aufbringen dieses Katalysators auf die Membran wird das erhaltene Produkt mit den üblichen Methoden in den einsatzbereiten Zustand verbracht, beispielsweise durch Tempern, bei hohen Temperaturen, die zur Versinterung führen, auch in speziellen Gasatmosphären, beispielsweise Inertgas, Sauerstoff oder Luft.

Die fertige, erfindungsgemäße Reaktionsmembran wird dann in die jeweilige Oxidationsreaktion eingesetzt, wobei ein sauerstoffhaltiges Fluid die Membran auf der Seite umströmt, die nicht mit Katalysator beschichtet ist. Als sauerstoffhaltiges Fluid wird dabei Luft oder Luft mit verändertem Sauerstoffanteil verwendet, wobei jeweils weitere Komponenten, die verdampfbar sein können oder nicht, vorhanden sein können. Der Druck, unter dem dieses Fluid steht, beträgt von 0,1 bis 100 bar, vorzugsweise 0,6 bis 50 bar, meist bevorzugt 0,6 bis 30 bar.

Auf der Seite der Membran, auf der der Oxidationskatalysator angebracht ist, befindet sich dann während der Reaktion das Gemisch, das die zu oxidierende Komponente enthält. Dieses Gemisch kann gasförmig, flüssig oder eine Mischung aus gasförmigen und flüssigen Komponenten sein. Dieses Gemisch befindet sich unter den gleichen Drücken wie das sauerstoffhaltige Fluid, nämlich 0,1 bis 100 bar, vorzugsweise 0,6 bis 50 bar, meist bevorzugt 0,6 bis 30 bar.

In einer Ausführungsform der Reaktionsmembran ist auf der dem sauerstoffhaltigen Fluid zugewandten Seite ein Redoxkatalysator aufgebracht, der die Reaktion von molekularem Sauerstoff zu Oxidionen katalysiert und damit den Eintritt des Sauerstoffs in die Membran erleichtert. Derartige Redoxkatalysatoren sind z.B. in Solid State Ionics 113 bis 115 (1998), S. 639 ff. beschrieben.

Die Oxidationsreaktion wird aufgrund der großen Wärmeentwicklung vorteilhafterweise so geführt, daß zwischen mehreren längs der Strömungsrichtung des Reaktionsgases angeordneten Zonen gekühlt wird. Dieser Kühlvorgang erfolgt auf die übliche, einem Fachmann bekannte Weise. Beispielsweise kann die Reaktion derart ausgeführt werden, daß zwischen mehreren Zonen, die längs der Strömungsrichtung der Reaktionsmischung angeordnet sind, ein Fluid eingeführt wird, wobei die Temperatur dieses Fluids unter der des in die Zone eintretenden Reaktionsgases liegt. So wird ein in vielen Fällen ausreichender Kühleffekt erreicht. Geeignete fluide Medien sind etwa Wasser oder Wasserdampf, Sauerstoff, Luft oder Luft mit verändertem Sauerstoffanteil, Stickstoff oder Ammoniak.

Ein Kühleffekt kann auch dadurch erreicht werden, daß auf einer Seite der Membran sich ein Medium befindet, das siedet. Durch den Siedevorgang läßt sich in vielen Fällen eine wirkungsvolle Kühlung der Membran erreichen. Möglich ist auch die Kühlung durch Wärmeaustausch zwischen einem oder beiden Teilströmen, die den Reaktor verlassen, und den entsprechenden Teilströmen, die in den Reaktor eintreten. Eine solche Ausführungsform hat den Vorteil, daß die in der Oxidationsreaktion frei werdende überschüssige Wärme zum Erhitzen des Eduktgemischs verwendet werden kann.

In einer bevorzugten Ausführungsform der vorliegenden Erfindung findet die Oxidationsreaktion in einem Rohrreaktor statt, wobei die erfindungsgemäße Reaktionsmembran natürlich die entsprechende Form aufweisen muß. Ein Teil der Rohre wird dabei zur Kühlung verwendet, nach den oben dargelegten Methoden. Vorzugsweise findet eine solche Kühlung in einem Rohrreaktor durch Verdampfen eines geeigneten Mediums statt.

Die erfindungsgemäße Reaktionsmembran kann in den üblichen Reaktoren aus den einem Fachmann bekannten Materialien verwendet werden. Beispiele für solche Materialien sind C-Stahl, Edelstahl oder Hastelloy. Besonders vorteilhaft ist es, wenn die Membran in einem Reaktor verwendet wird, der vollständig aus keramischen Materialien besteht. Es ergeben sich hierbei keine Probleme, die durch stark unterschiedliche Ausdehnungskoeffizienten des Reaktormaterials und des Membranmaterials entstehen.

Die erfindungsgemäßen Reaktionsmembranen mit dem aufgebrachten Oxidationskatalysator lassen sich vorteilhafterweise bei Oxidationen im Temperaturbereich von 200 bis 600, vorzugsweise 300 bis 500°C einsetzen. Dies ist mit den bisherigen, im Stand der Technik bekannten Membranen nicht möglich. Die Reaktionsmembranen nach der vorliegenden Erfindung eignen sich jedoch selbstverständlich auch dazu, in Oxidationen bei höheren Temperaturen als den angegebenen verwendet zu werden, etwa bis 1200°C. Die erfindungsgemäßen Reaktionsmembranen können bei sämtlichen Oxidationsreaktionen verwendet werden, die im Temperaturbereich von 200 bis 1200°C ablaufen.

Die vorliegende Erfindung wird nun in dem nachfolgenden Beispiel erläutert:

### Beispiel

### Oxidation von n-Butan in einem Oxidations-Membranreaktor

Ein poröses Rohr mit einem Außendurchmesser von 10 mm, einem Innendurchmesser von 7 mm und einer Länge von 20 cm aus Magnesiumoxid mit einer mittleren Porengröße von 2 µm wurde als Träger verwendet. Ein feindisperses Pulver der Zusammensetzung Sr_{0,5}Ca_{0,5}MnO_{3-δ} wurde als wäßriger Schlicker aufbereitet. Durch Tauchbeschichtung wurde die Innenseite des vorgenannten porösen Rohres beschichtet. Die thermische Behandlung bei 1200°C führte zu einer porösen Schicht mit einer Schichtdicke von 20 µm und einer offenen Porosität von 45 % bei einer mittleren Porengröße von 0,7 µm. Auf diese Schicht wurde mittels Sol-Gel-Technik eine dünne Schicht von Sr_{0,5}Ca_{0,5}MnO_{3-δ} durch Tauchbeschichtung aufgebracht. Die thermische Behandlung führte zu einer dichten Schicht mit einer Schichtdicke von 1 bis 2 µm.

Auf diese Schicht im Rohrinnern wurde mittels Tauchbeschichtung eine Suspension, bestehend aus 235 g (VO)₂P₂O₇ (hergestellt nach EP 39 537) und bestehend aus Teilchen mit einer durchschnittlichen Teilchengröße von 5 µm, 800 ml Wasser und 100 ml Glycerin als Haftmittel, in der Art aufgebracht, daß eine ca. 50 µm dicke Katalysatorschicht entstand. Zur Trocknung der aufgebrachten Katalysatorschicht und zur Entfernung des noch anhaftenden Glycerins wurde das beschichtete Rohr bei 110°C 5 h lang und anschließend bei 250°C 10 h lang erhitzt.

Durch das Rohr ließ man 10 Nl/h n-Butan strömen. Die Außenseite des Rohres wurde mit 50 Nl/h Luft umströmt. Beide Gasströme wurden vor Eintritt in den Reaktor auf 420°C vorgewärmt. Die Reaktionsmembran wurde ebenfalls auf 420°C temperiert.
Das entstandene Reaktionsgemisch wurde gaschromatographisch analysiert. Es wurde eine Maleinsäureanhydrid-Bildung von 0,1 Mol Maleinsäureanhydrid pro Stunde und pro m² Membranfläche gefunden.

## Patentansprüche

1. Reaktionsmembran enthaltend einen selektiven Oxidationskatalysator auf einer gemischtleitenden, oxidionenselektiven keramischen Membran der Zusammensetzung (Sr₁₋ₓCaₓ)_{1-y}A_{y}Mn_{1-z}B_{z}O_{3-δ}, wobei
A für Ba, Pb, Na, K, Y, ein Element der Lanthanidengruppe oder eine Kombination dieser Elemente steht,
B für Mg, Al, Ga, In, Sn, ein Element der 3d- oder der 4d-Periode oder eine Kombination dieser Elemente steht,
x eine Zahl von 0,2 bis 0,8 ist,
y eine Zahl von 0 bis 0,4 ist,
z eine Zahl von 0 bis 0,6 ist und
δ eine Zahl ist, die sich in Abhängigkeit von x, y und z aus der Einhaltung des Elektroneutralitätsprinzips ergibt.

2. Reaktionsmembran nach Anspruch 1, **dadurch gekennzeichnet, daß** diese flach ist oder Rohrgeometrie mit Innendurchmessern von 4 bis 100 mm aufweist.

3. Reaktionsmembran nach Anspruch 1 oder 2, **dadurch gekennzeichnet, daß** der Katalysator als poröse Schicht vorliegt oder selbst gemischtleitende, oxidionenselektive Eigenschaften aufweist.

4. Reaktionsmembran nach einem der Ansprüche 1 bis 3, **dadurch gekennzeichnet, daß** der Katalysator in einer Schichtdicke von 10 Å bis 1 mm auf der Membran angebracht ist.

5. Reaktionsmembran nach einem der Ansprüche 1 bis 4, **dadurch gekennzeichnet, daß** zwischen der Membran und dem Oxidationskatalysator eine oxidische Zwischenschicht mit einer Dicke von 10 Å bis 10 µm angebracht ist.

6. Verwendung einer Reaktionsmembran nach einem der Ansprüche 1 bis 5 in Oxidationsreaktionen von Kohlenwasserstoffen unter Anwesenheit von Sauerstoff.

7. Verwendung nach Anspruch 6, **dadurch gekennzeichnet, daß** die Oxidationsreaktion bei Temperaturen von 200 bis 1200°C, vorzugsweise 200 bis 600°C, meist bevorzugt 300 bis 500°C durchgeführt wird.

8. Verwendung nach Anspruch 6 oder 7, **dadurch gekennzeichnet, daß** die Oxidationsreaktion eine Ammonoxidation, die Synthese von Maleinsäureanhydrid oder Phthalsäureanhydrid aus linearen C₄-Kohlenwasserstoffen, vorzugsweise n-Butan oder n-Xylol, die Synthese von (Meth)acrolein oder (Meth)acrylsäure aus dem entsprechenden Kohlenwasserstoff oder eine oxidative Dehydrierung eines Alkans zum entsprechenden Alken oder eine oxidative Dehydrierung eines Alkohols zum entsprechenden Aldehyd ist.

9. Verwendung nach einem der Ansprüche 6 bis 8, **dadurch gekennzeichnet, daß** Sauerstoff in Form von Luft oder Luft mit verändertem Sauerstoffanteil, gegebenenfalls in Anwesenheit einer weiteren verdampfbaren fluiden Komponente, eingesetzt wird.

10. Verwendung nach einem der Ansprüche 6 bis 9, **dadurch gekennzeichnet, daß** der Druck auf der Seite der Membran, auf der der Katalysator angebracht ist, 0,1 bis 100 bar, vorzugsweise 0,6 bis 50 bar, meist-bevorzugt 0,6 bis 30 bar, und der Druck auf der Seite der Membran, auf der sich der Katalysator nicht befindet, 0,1 bis 100 bar, vorzugsweise 0,6 bis 50 bar, meistbevorzugt 0,6 bis 30 bar, beträgt.

## Claims

1. A reactor membrane comprising a selective oxidation catalyst on a mixed conducting, oxide ion selective ceramic membrane of the composition (Sr₁₋ₓCaₓ)_{1-y}A_{y}Mn_{1-z}B_{z}O_{3-δ}, where
A is Ba, Pb, Na, K, Y, an element of the lanthanide group or a combination thereof,
B is Mg, Al, Ga, In, Sn, an element of the 3d or 4d period or a combination thereof,
x is from 0.2 to 0.8,
y is from 0 to 0.4,
z is from 0 to 0.6, and
δ is a number, dependent on x, y and z, that renders the composition charge neutral.

2. The reactor membrane according to claim 1 that is flat or has a tube geometry with internal diameters from 4 to 100 mm.

3. The reactor membrane according to claim 1 or 2 wherein the catalyst is a porous layer or itself has mixed conducting, oxide ion selective properties.

4. The reactor membrane according to any of claims 1 to 3 wherein the catalyst has been applied to said membrane in a layer thickness from 10 Å to 1 mm.

5. The reactor membrane according to any of claims 1 to 4 comprising an oxidic interlayer from 10 Å to 10 µm in thickness between said membrane and said oxidation catalyst.

6. The use of a reactor membrane according to any of claims 1 to 5 in oxidation reactions of hydrocarbons in the presence of oxygen.

7. The use according to claim 6 wherein the oxidation reaction is carried out at from 200 to 1200°C, preferably from 200 to 600°C, most preferably from 300 to 500°C.

8. The use according to claim 6 or 7 wherein the oxidation reaction is an ammoxidation, the synthesis of maleic anhydride or phthalic anhydride from linear C₄ hydrocarbons, preferably n-butane or n-xylene, the synthesis of (meth)acrolein or (meth)acrylic acid from the corresponding hydrocarbon or an oxidative dehydrogenation of an alkane to the corresponding alkene or an oxidative dehydrogenation of an alcohol to the corresponding aldehyde.

9. The use according to any of claims 6 to 8 wherein oxygen is used in the form of air or air having a modified oxygen fraction, in the presence or absence of a further vaporizable fluid component.

10. The use according to any of claims 6 to 9 wherein the pressure on the side of said membrane to which said catalyst has been applied is from 0.1 to 100 bar, preferably from 0.6 to 50 bar, most preferably from 0.6 to 30 bar, and the pressure on the side of said membrane on which said catalyst is not located is from 0.1 to 100 bar, preferably from 0.6 to 50 bar, most preferably from 0.6 to 30 bar.

## Revendications

1. Membrane réactive qui contient un catalyseur sélectif d'oxydation sur une membrane céramique à conduction mixte et sélective vis-à-vis des ions oxydés, de composition (Sr₁₋ₓCaₓ)_{1-y}A_{y}Mn_{1-z}B_{z}O_{3-δ}, dans laquelle :
A représente Ba, Pb, Na, K, Y, un élément du groupe des lanthanides ou une combinaison de ces éléments,
B représente Mg, Al, Ga, In, Sn, un élément du groupe 3d ou du groupe 4d du tableau périodique ou une combinaison de ces éléments,
x représente un nombre compris entre 0,2 et 0,8,
y représente un nombre compris entre 0 et 0,4,
z représente un nombre compris entre 0 et 0,6 et
δ est un nombre qui s'obtient en fonction de x, y et z en respectant la loi de neutralité électronique.

2. Membrane réactive selon la revendication 1, **caractérisée en ce qu'**elle est plate ou présente une géométrie tubulaire d'un diamètre intérieur de 4 à 100 mm.

3. Membrane réactive selon les revendications 1 ou 2, **caractérisée en ce que** le catalyseur se présente en couche poreuse ou présente lui-même des propriétés de conduction mixte et de sélection vis-à-vis des ions oxydés.

4. Membrane réactive selon l'une des revendications 1 à 3, **caractérisée en ce que** le catalyseur est appliqué sur la membrane en couche d'une épaisseur de 10 Å à 1 mm.

5. Membrane réactive selon l'une des revendications 1 à 4, **caractérisée en ce qu'**une couche intermédiaire oxydée d'une épaisseur de 10 Å à 10 µm est appliquée entre la membrane et le catalyseur d'oxydation.

6. Utilisation d'une membrane réactive selon l'une des revendications 1 à 5 dans des réactions d'oxydation d'hydrocarbures en présence d'oxygène.

7. Utilisation selon la revendication 6, **caractérisée en ce que** la réaction d'oxydation est réalisée à des températures de 200 à 1 200°C, de préférence de 200 à 600°C et de façon particulièrement préférée de 300 à 500°C.

8. Utilisation selon les revendications 6 ou 7, **caractérisée en ce que** la réaction d'oxydation est une ammonoxydation, la synthèse d'anhydride maléique ou d'anhydride phtalique à partir d'hydrocarbures linéaires en C₄, de préférence de n-butane ou de n-xylène, la synthèse de (méth)acroléine ou d'acide (méth)acrylique à partir de l'hydrocarbure concerné, la déshydrogénation oxydative d'un alcane en l'alcène correspondant ou la déshydrogénation oxydative d'un alcool en l'alhéhyde correspondant.

9. Utilisation selon l'une des revendications 6 à 8, **caractérisée en ce que** l'oxygène est utilisé sous la forme d'air ou d'air dont la teneur en oxygène est modifiée, éventuellement en présence d'un autre composant fluide et vaporisable.

10. Utilisation selon l'une des revendications 6 à 9, **caractérisée en ce que** sur le côté de la membrane sur laquelle le catalyseur est appliqué, la pression est de 0,1 à 100 bars, de préférence de 0,6 à 50 bars et de façon particulièrement préférable de 0,6 à 30 bars, et **en ce que** sur le côté de la membrane sur lequel le catalyseur ne se trouve pas, la pression est comprise entre 0,1 et 100 bars, de préférence entre 0,6 et 50 bars et de la façon la plus préférable entre 0,6 et 30 bars.
